# EUROPÄISCHE PATENTSCHRIFT

(11) **EP 2 349 353 B1**
(45) Veröffentlichungstag und Bekanntmachung des Hinweises auf die Patenterteilung: **29.11.2017**
(21) Anmeldenummer: 09759649.8
(22) Anmeldetag: 22.10.2009
(51) Int. Cl.: A61L 2/00, A61L 2/08

(54) **VERFAHREN UND ANORDNUNG ZUM STERILISIEREN, INSBESONDERE ZUM STERILISIEREN EINES ADSORBERS**
METHOD AND ARRANGEMENT FOR STERILIZING, IN PARTICULAR FOR STERILIZING AN ADSORBER
PROCÉDÉ ET DISPOSITIF DE STÉRILISATION, EN PARTICULIER POUR LA STÉRILISATION D'UN ADSORBANT

(30) Priorität: 24.10.2008 DE 102008053131
(43) Veröffentlichungstag der Anmeldung: 03.08.2011
(73) Patentinhaber: Fresenius Medical Care Deutschland GmbH, 61352 Bad Homburg v.d.H. (DE)
(72) Erfinder: RAHN, Holger, 66606 St. Wendel (DE); LEINENBACH, Hans-Peter, Dr., A-3500 Krems-Rehberg (AT); KUHN, Stefan, 66538 Neunkirchen (DE); GERNER, Franz-Josef, 66606 St. Wendel (DE)
(74) Vertreter: Oppermann, Frank
(86) Internationale Anmeldenummer: PCT/EP2009/007565
(87) Internationale Veröffentlichungsnummer: WO 2010/046107

(56) Entgegenhaltungen:
- EP-A- 0 507 245
- EP-A- 1 902 740
- DE-U1- 29 801 590
- US-A1- 2005 142 542

## Beschreibung

Die Erfindung betrifft ein Verfahren zum Sterilisieren eines in einem Behälter bereitzustellenden Adsorbens. Darüber hinaus betrifft die Erfindung eine Anordnung zur Durchführung des Verfahrens.

Es sind verschiedene Behandlungsverfahren bekannt, die von einem Adsorber Gebrauch machen. Adsorbertherapien sind innovative Behandlungsverfahren, die krankheitsverursachende Stoffe außerhalb des Körpers aus dem Blut des Patienten entfernen. Beispielsweise kann mit der LDL-Apherese LDL-Cholesterin aus dem Vollblut entfernt werden. Die Immunadsorbtion beispielsweise erlaubt die Entfernung von Antikörpern aus dem Blut des Patienten.

Die bekannten Adsorber verfügen über ein Adsorbergehäuse, das mit einem Adsorbens dicht gepackt ist. Das Adsorbergehäuse ist im Allgemeinen ein hohlzylindrisches Gehäuse, das an der Ober- und Unterseite verschlossen ist. An der Oberseite weist das Gehäuse einen Zulauf und an der Unterseite einen Ablauf auf. Ein vor dem Ablauf angeordnetes Sieb verhindert, dass Adsorbens aus dem Gehäuse austritt.

Die Sterilisation des mit dem Adsorbens gefüllten Adsorbergehäuses erfolgt mittels einer energiereichen, ionisierenden Strahlung, beispielsweise α-, β- oder γ-Strahlung, wobei die Sterilisation mit β-Strahlung bevorzugt wird. Diese energiereiche Strahlung kann aber bei einer zu hohen Intensität das Adsorbens schädigen. Es ist daher wichtig, dass die Dosis der Strahlung für die Sterilisation ausreichend ist, die Strahlungsdosis aber nicht zu hoch ist, um Schädigungen des Adsorbens zu verhindern. Durch Absorbtion der β-Strahlung im Adsorbens verringert sich die Strahlungsintensität, wobei mit zunehmender Länge des Strahlenweges die Strahlungsintensität beträchtlich abnimmt. Der Energieverlust der Teilchenstrahlung steigt pro Weglängeneinheit während des Weges, den die Teilchen zurücklegen, zunächst an, um dann nach einem Maximum abrupt abzufallen. Der Energieverlust pro Weglängeneinheit wird durch die Bragg-Kurve beschrieben.

In der Praxis erweist sich als nachteilig, dass die bekannten Adsorbergehäuse aufgrund ihrer geometrischen Abmessungen eine relativ hohe Bestrahlungsdosis erfordern, um sicherzustellen, dass das gesamte Adsorbens im Inneren des Gehäuses sterilisiert wird. Aufgrund einer zu hohen Bestrahlungsdosis bei einer Schichttiefe um das Maximum der Bragg-Kurve besteht aber die Gefahr einer Schädigung zumindest eines Teils des Adsorbens. Dieses Problem wird bisher dadurch gelöst, dass das Adsorbens getrocknet wird, da die Eindringtiefe der ionisierenden β-Strahlung in trockenem Material größer als in feuchtem Material ist. Allerdings erfordert dieses Verfahren den zusätzlichen Verfahrensschritt der Trocknung, der auch mit einem zusätzlichen Energieverbrauch verbunden ist.

Aus der GB 655 198 ist ein Verfahren und eine Vorrichtung zum Bestrahlen von dünnen Flüssigkeitsfilmen bekannt. Die bekannte Vorrichtung umfasst einen rotierenden Zylinder, an dessen Wandung die zu bestrahlende Flüssigkeit fließt. Das Prinzip der Strahlungssterilisation dünner Schichten ist auch aus der WO 2004/075931 A2 bekannt.

Die EP 1 902 740 A beschreibt ein Blutbeutel-System, das einen Beutel umfasst, in dem Blutbestandteile einer Bestrahlung mit UV-Licht ausgesetzt werden sollen. Die Strahlendosis der UV-Strahlung soll zwischen 0,01 und 2 J/cm² liegen. Es handelt sich bei der UV-Strahlung um eine Strahlung mit einer relativ niedrigen Strahlendosis, da ansonsten die Zerstörung der Blutbestandteile, insbesondere Leukozyten, die Folge wäre. Der Sammelbeutel für die Blutbestandteile ist über eine Leitung mit einem Lagerbeutel verbunden, in den die Blutbestandteile überführt werden können.

Aus der US 2005/0142542 A1 ist eine Anordnung zur Reduzierung der Konzentration von Bestandteilen in biologischen Flüssigkeiten bekannt, die einen Adsorbensbehälter aufweist, an den mit einer Leitung ein weiterer Behälter angeschlossen ist.

Die oben genannten Probleme stellen sich nicht nur bei der Sterilisiation eines Adsorbens in einem Adsorbergehäuse, sondern auch bei der Sterilisation anderer strahlungsempfindlicher Materialien, die in einem Behälter bereitgestellt werden sollen, beispielsweise bei Medikamenten zur Infusion, aber auch bei Nahrungsmitteln.

Der Erfindung liegt daher die Aufgabe zugrunde, ein vereinfachtes Verfahren zum Sterilisieren eines Adsorbens anzugeben, bei dem nicht die Gefahr der Schädigung Adsorbens besteht.

Eine weitere Aufgabe der Erfindung ist, eine Anordnung zu schaffen, mit der das Sterilisieren mit dem erfindungsgemäßen Verfahren vereinfacht wird, ohne dass die Gefahr der Schädigung des Adsorbens besteht.

Die Lösung dieser Aufgaben erfolgt erfindungsgemäß mit den Merkmalen der Patentansprüche 1 und 11. Vorteilhafte Ausführungsformen der Erfindung sind Gegenstand der Unteransprüche.

Das erfindungsgemäße Verfahren zum Sterilisieren eines Adsorbens, das in einem Behälter bereitgestellt werden soll, beruht darauf, dass ein separates Behältnis, das mit dem Behälter, in dem das Adsorbens sterilisiert werden soll, ein geschlossenes System bildet, mit dem Adsorbens befüllt wird, wobei das separate Behältnis zum Sterilisieren derart bemessen ist, dass sich das Adsorbens in dem Behältnis in einer dünnen Schicht verteilt. Zumindest das separate Behältnis mit dem Adsorbens wird dann mit einer energiereichen Strahlung bestrahlt. Vorzugsweise wird das gesamte geschlossene System bestrahlt. Da der Behälter, in dem das Adsorbens sterilisiert werden soll und das Behältnis zum Sterilisieren ein geschlossenes System bilden, kann das sterilisierte Adsorbens aseptisch in den Behälter überführt werden, in dem das Material bereitgestellt werden soll. Dies hat den Vorteil, dass eine aseptische Befüllung des Behälters, in dem das Adsorbens bereitgestellt werden soll, in einer nicht sterilen Umgebung nicht erforderlich ist. Dadurch wird die Sterilisation mit dem erfindungsgemäßen Verfahren vereinfacht.

Als besonders vorteilhaft erweist sich das erfindungsgemäße Verfahren und die erfindungsgemäße Vorrichtung zum Sterilisieren eines Adsorbers, der ein mit einem Adsorbens zu befüllendes Adsorbergehäuse aufweist. Das erfindungsgemäße Verfahren und die erfindungsgemäße Vorrichtung beruhen darauf, dass nicht das mit dem Adsorbens gefüllte Adsorbergehäuse, sondern das Adsorbens ohne das Adsorbergehäuse in einem separaten Behältnis sterilisiert wird, das derart bemessen ist, dass sich das Adsorbens in dem Behältnis in einer dünnen Schicht verteilt. Dabei ist unter einer dünnen Schicht eine Schicht mit einer Schichtdicke von bis zu 30 mm, vorzugsweise kleiner 20 mm, besonders bevorzugt zwischen 5 und 10 mm zu verstehen. Hierfür ist jedes Behältnis geeignet, das im Verhältnis zum Volumen des Adsorbens ausreichende Abmessungen hat. Auch ein flaches Behältnis ist ausreichend, dessen Höhe im Verhältnis zur Breite und Länge sehr klein ist.

Da das Adsorbens in dem Behältnis in einer dünnen Schicht verteilt ist, verändert sich die Strahlungsdosis innerhalb der dünnen Schicht nur unwesentlich. Aufgrund der gleichmäßigen Verteilung der Strahlungsdosis kann das Material mit einer Strahlungsdosis bestrahlt werden, die grundsätzlich zur Sterilisation an der Oberfläche erforderlich ist. Dies stellt einerseits sicher, dass das gesamte Material mit einer ausreichenden Dosis bestrahlt wird, andererseits besteht nicht die Gefahr einer Schädigung zumindest eines Teils des Adsorbens aufgrund einer zu hohen Strahlungsdosis in einzelnen Bereichen der tieferen Schichten.

Bei einer besonders bevorzugten Ausführungsform ist das Behältnis zur Aufnahme des Adsorbens ein Beutel, in dem sich das Adsorbens in einer dünnen Schicht verteilt, wenn der Beutel auf einer Ebene flach aufliegt. Vorzugsweise ist der Beutel ein aus zwei übereinander liegenden Folien oder ein aus einer Schlauchfolie gefertigter Folienbeutel. Derartige Beutel zur Aufnahme von medizinischen Flüssigkeiten sind allgemein bekannt. Ein Beutel an Stelle eines starren Behälters hat auch den Vorteil, dass sich das Adsorbens einfach aus dem Beutel überführen lässt. Dadurch kann der Beutel einfach und vollständig entleert werden.

Da sich das Adsorbens in dem Behältnis in einer dünnen Schicht verteilt, variiert die Strahlendosis innerhalb der dünnen Schicht nur geringfügig. Das Behältnis wird mit einer energiereichen, insbesondere ionisierenden Strahlung, beispielweise einer α-, β- oder γ-Strahlung, vorzugsweise einer β-Strahlung, bestrahlt. Anschließend wird das Adsorbens aus dem Behältnis in das Adsorbergehäuse überführt. Es ist möglich, nur das Behältnis mit dem Adsorbens zu bestrahlen. Vorzugsweise wird aber die gesamte Anordnung bestrahlt, die das Adsorbergehäuse und das Behältnis zur Aufnahme des Adsorbens als geschlossenes System umfasst.

Beim Überführen des Adsorbens in das Adsorbergehäuse wird die in dem Adsorbergehäuse befindliche Luft und/oder überschüssige Flüssigkeit vorzugsweise aus dem Adsorbergehäuse abgeführt.

Bei einer bevorzugten Ausführungsform wird Luft und/oder überschüssige Flüssigkeit in ein oder mehrere separate Behältnisse überführt. Dabei ist aber sicherzustellen, dass das Adsorbergehäuse und das Behältnis zur Aufnahme des Adsorbens sowie weitere Behältnisse wieder ein geschlossenes System bilden. Das Behältnis zur Aufnahme von Luft und/oder Flüssigkeit, bei dem es sich vorzugsweise wieder um einen Beutel handelt, ist vorzugsweise mit dem Adsorbergehäuse über eine oder mehrere Schlauchleitungen verbunden.

Bei einer alternativen Ausführungsform wird ein geschlossenes System mit einer oder mehreren sterilen Barrieren, insbesondere hydrophoben, d.h. für Luft durchlässigen, für Flüssigkeit aber undurchlässigen sterilen Membranen geschaffen, die ein oder mehrere Entlüftungsöffnungen des Adsorbergehäuses steril verschließen. Bei dieser Ausführungsform kann allerdings das Adsorbergehäuse nur entlüftet, nicht aber überschüssige Flüssigkeit abgeführt werden.

Bei einer weiteren bevorzugten Ausführungsform wird die in dem Adsorbergehäuse befindliche Luft und/oder überschüssige Flüssigkeit nicht in ein oder mehrere separate Behältnisse überführt, sondern in den entleerten Behälter zur Aufnahme des Adsorbens.

Für den Gebrauch des Adsorbergehäuses werden die Schlauchleitungen abgetrennt. Die Schlauchleitungen können mit Absperrorganen unterschiedlicher Ausbildung verschlossen werden. Es ist aber auch möglich, die Schlauchleitungen steril abzuschweißen. Grundsätzlich sind alle bekannten Vorrichtungen und Verfahren möglich, mit denen sich die Schlauchleitungen steril abtrennen oder abschließen lassen.

Bei einer besonders bevorzugten Ausführungsform wird die in dem Adsorbergehäuse befindliche Luft und/oder überschüssige Flüssigkeit an zwei gegenüberliegenden Seiten des Adsorbergehäuses, vorzugsweise an der Ober- und Unterseite des in der Gebrauchslage befindlichen Adsorbergehäuses abgeführt. Dabei kann die Luft und/oder Flüssigkeit in ein separates Behältnis oder zwei separate Behältnisse überführt werden. Die Behältnisse, die vorzugsweise Beutel sind, können separate Beutel oder Kammern eines Mehrkammerbeutels sein. Eine besonders bevorzugte Ausführungsform sieht vor, dass der Beutel zur Aufnahme des Adsorbens und die beiden Beutel zur Aufnahme von Luft und/oder Flüssigkeit einen Mehrkammerbeutel, d.h. einen Dreikammer-Beutel bilden.

Das Behältnis zur Aufnahme des Adsorbens weist vorzugsweise einen Einlass auf, über den das Behältnis mit dem Adsorbens befüllt werden kann. An den Einlass kann eine Schlauchleitung oder ein Konnektor zum Anschluss einer Schlauchleitung angeschlossen sein.

Das Behältnis kann mit einem flüssigen oder trockenen, aber rieselfähigen Material, insbesondere Adsorbens, befüllt werden. Nach der Sterilisation kann das flüssige Material, insbesondere das Adsorbens als Suspension in den Behälter, insbesondere das Adsorbergehäuse überführt werden. Das trockene, insbesondere pulverförmige Material kann nach dem Sterilisieren entweder in trockenem Zustand überführt werden oder erst mit einer Flüssigkeit in Lösung oder Suspension gebracht und dann in flüssigem Zustand überführt werden. Diese Flüssigkeit kann wieder in einem Behältnis, insbesondere in einem Beutel bereitgestellt werden.

Eine weitere besonders bevorzugte Ausführungsform sieht eine Spülung des Behältnisses zur Aufnahme des Adsorbens vor. Bei dieser Ausführungsform umfasst die erfindungsgemäße Anordnung ein weiteres Behältnis zur Aufnahme einer Spülflüssigkeit, die in das entleerte Behältnis zur Aufnahme des Adsorbens überführt wird, nachdem das Adsorbens in das Adsorbergehäuse überführt worden ist. Nach dem Spülen des Behältnisses wird die Spülflüssigkeit dann zusammen mit der Restmenge an Adsorbens in das Adsorbergehäuse überführt, wobei überschüssige Flüssigkeit und/oder Luft wieder aus dem Adsorbergehäuse abgeführt wird.

Im Folgenden werden verschiedene Ausführungsbeispiele der Erfindung unter Bezugnahme auf die Zeichnungen näher erläutert.

Es zeigen:
- Fig. 1: Ein erstes Ausführungsbeispiel einer erfindungsgemäßen Anordnung zum Sterilisieren eines Adsorbers in stark vereinfachter schematischer Darstellung, bei der in dem Adsorbergehäuse befindliche Luft und/oder überschüssige Flüssigkeit in zwei Leerbeutel überführt wird,
- Fig. 2: ein zweites Ausführungsbeispiel der erfindungsgemäßen Anordnung, bei der in dem Adsorbergehäuse befindliche Luft und/oder überschüssige Flüssigkeit in einen Leerbeutel überführt wird,
- Fig. 3: ein weiteres Ausführungsbeispiel der erfindungsgemäßen Anordnung, bei der der Beutel zur Aufnahme des Adsorbens und die Beutel zur Aufnahme der Luft und/oder Flüssigkeit einen Mehrkammer-Beutel bilden,
- Fig. 4: ein weiteres Ausführungsbeispiel der erfindungsgemäßen Anordnung, bei der in dem Adsorbergehäuse befindliche Luft in die Umgebung abgeführt wird,
- Fig. 5: ein weiteres Ausführungsbeispiel der erfindungsgemäßen Anordnung, die eine Spülung des Beutels zur Aufnahme des Adsorbens erlaubt und
- Fig. 6: eine Ausführungsform der erfindungsgemäßen Anordnung, bei der in dem Adsorbergehäuse befindliche Luft in den Beutel zur Aufnahme des Adsorbens überführt wird.

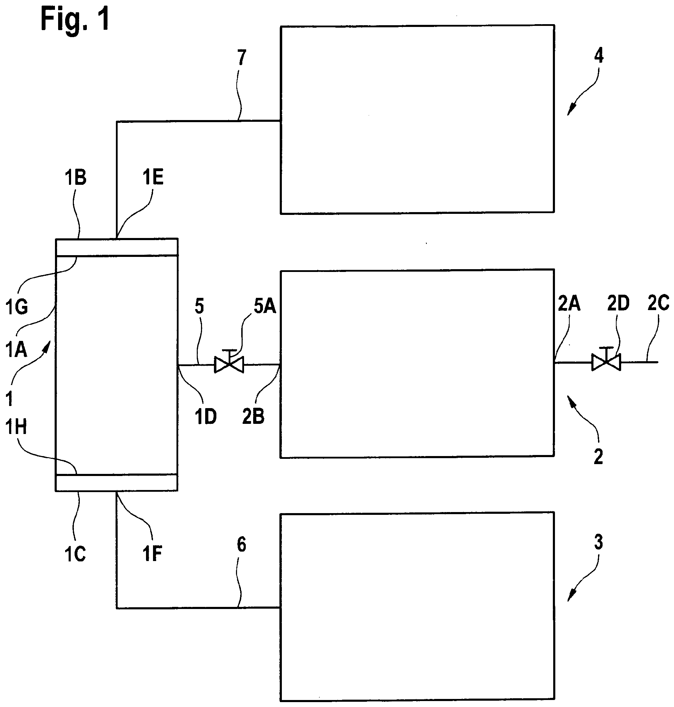

Fig. 1 zeigt ein erstes Ausführungsbeispiel der erfindungsgemäßen Anordnung zum Sterilisieren eines Adsorbers in stark vereinfachter schematischer Darstellung.

Die erfindungsgemäße Anordnung umfasst den Adsorber 1, ein Behältnis 2 zur Aufnahme eines Adsorbens in Form einer Suspension sowie ein erstes und ein zweites Behältnis 3, 4 zur Aufnahme von in dem Adsorbergehäuse befindlicher Luft und/oder überflüssiger Flüssigkeit. Bei den Behältnissen zur Aufnahme eines Adsorbens bzw. Luft und/oder Flüssigkeit handelt es sich um einen konventionellen Beutel, die in der Medizintechnik allgemein Verwendung finden. Derartige Beutel werden aus zwei übereinander liegenden Folien gefertigt, die im Randbereich mit einer umlaufenden Schweißnaht verschweißt sind. Die Beutel können aber auch aus einer Schlauchfolie gefertigt sein, die an ihren Enden mit einer Schweißnaht verschweißt ist. Da derartige Beutel zum Stand der Technik gehören, erübrigt sich eine weitere Beschreibung.

Für die Erfindung entscheidend ist, dass der Beutel zur Aufnahme des Adsorbens im Verhältnis zum Volumen des Adsorbens eine ausreichende Größe hat. Wenn der Beutel flach auf einer Ebene aufliegt, verteilt sich das Adsorbens in dem Beutel in einer dünnen Schicht.

Der Adsorber 1 ist ein konventioneller Adsorber, der mit dem Adsorbens zu befüllen ist. Der Adsorber verfügt über ein hohlzylindrisches Adsorbergehäuse 1A, das an den Enden mit einem Deckelteil 1B und einem Bodenteil 1C verschlossen ist. Ein derartiger Adsorber ist beispielsweise aus der EP 0 507 245 B1 bekannt. Da der Adsorber als solches zum Stand der Technik gehört, werden hier nur die für die Erfindung relevanten Teile beschrieben. Beispielsweise sind der Ein- und Auslass des Adsorbers zum Zu- und Abführen des zu behandelnden Bluts nicht gezeigt.

Der Beutel 2 zur Aufnahme des Adsorbens weist eine Einlassöffnung 2A zum Befüllen des Beutels 2 mit einem Adsorbens in Form einer Suspension und eine Auslassöffnung 2B zum Entleeren des Beutels auf. Zu der Einlassöffnung 2A führt eine Schlauchleitung 2C, die mit einem Absperrorgan 2D verschlossen ist. An die Auslassöffnung 2B ist das eine Ende einer Schlauchleitung 5 angeschlossen, dessen anderes Ende an eine Einlassöffnung 1D des Adsorbergehäuses 1A angeschlossen. Zum Öffnen und Schließen der Schlauchleitung 5 ist ein Absperrorgan 5A vorgesehen. Es ist aber grundsätzlich auch möglich, den Beutel 2 über die Leitung 5 zu befüllen und danach den Beutel mit dem Adsorbergehäuse zu verbinden. Bei dieser Ausführungsform kann die Leitung 2 mit der Einlassöffnung 2A entfallen.

Das Adsorbergehäuse 1A weist an dem Deckelteil 1B eine obere Entlüftungsöffnung 1E und an dem Bodenteil 1C eine untere Entlüftungsöffnung 1F auf. Unterhalb der oberen Entlüftungsöffnung 1E ist in dem hohlzylindrischen Absorbergehäuse 1A ein oberes Filtersieb 1G und oberhalb der unteren Entlüftungsöffnung 1F ein unteres Filtersieb 1H angeordnet, die das Adsorbens zurückhalten sollen. An der unteren Entlüftungsöffnung 1F ist über eine Schlauchleitung 6 der erste Beutel 3 zur Aufnahme von Luft und/oder Flüssigkeit, und an der oberen Entlüftungsöffnung 1E ist über eine Schlauchleitung 7 der zweite Beutel 4 zur Aufnahme von Luft und/oder Flüssigkeit angeschlossen.

Da es hier nur auf das Prinzip ankommt, sind der besseren Übersichtlichkeit halber die dem Fachmann bekannten Teile zum Anschluss der Schlauchleitungen an die Beutel nicht dargestellt.

Nachfolgend wird das erfindungsgemäße Verfahren am Beispiel des Verfahrens zum Sterilisieren eines Adsorbers unter Verwendung der erfindungsgemäßen Anordnung beschrieben.

Zunächst wird das Absperrorgan 2D an der Schlauchleitung 2C zum Zuführen des Adsorbens geöffnet, und das Adsorbens wird aus einem nicht dargestellten Reservoir über die Schlauchleitung 2C in den Beutel 2 geleitet. Dabei liegt der Beutel 2 flach auf einer Ebene auf, so dass sich das Adsorbens als dünner Film in dem Beutel verteilt. Anschließend wird das Absperrorgan 2D geschlossen.

Die gesamte Anordnung wird nunmehr mit einer ionisierenden Strahlung, beispielsweise einer α-, β- oder γ-Strahlung, vorzugsweise einer β-Strahlung bestrahlt. Es ist aber auch möglich, nur den Beutel 2 mit dem Adsorbens zu bestrahlen. Dabei ist die Bestrahlungsdosis so einzustellen, dass die Dosis für die Sterilistaion geeignet ist. Da sich die Strahlungsdosis innerhalb der dünnen Schicht in dem Beutel kaum verändert, braucht die Bestrahlungsdosis nicht höher oder geringer eingestellt zu werden, als die Dosis, die für eine Sterilisation an der Oberfläche im Allgemeinen ausreichend ist. Das Adsorbergehäuse 1A kann nunmehr befüllt werden. Nach der Sterilisation liegt noch ein geschlossenes System vor, da die einzelnen Behältnisse noch mit den Schlauchleitungen verbunden sind. Das Adsorbens kann somit in dem geschlossenen System über die Schlauchleitungen ohne die Notwendigkeit einer aseptischen Umgebung von dem Beutel 2 in das Adsorbergehäuse 1A überführt werden. Dabei ist die Sterilität gewahrt.

Nach dem Bestrahlen wird das Absperrorgan 5A an der Schlauchleitung 5 geöffnet. Das in dem Beutel 2 befindliche Adsorbens wird jetzt in das Adsorbergehäuse 1A überführt. Dies kann dadurch erfolgen, dass der Beutel 2 über dem Adsorbergehäuse angeordnet wird, so dass die Suspension in das Adsorbergehäuse aufgrund der Schwerkraft fließt. Der Beutel 2 kann zum Überführen der Suspension aber auch zusammengequetscht oder die Schlauchleitung in eine Schlauchpumpe eingelegt werden. Dies empfiehlt sich ohnehin zum vollständigen Entleeren des Beutels. Nachdem der Beutel entleert ist, wird das Absperrorgan 5A wieder geschlossen.

Die in dem Adsorbergehäuse 1A befindliche Luft sowie überschüssige Flüssigkeit strömt beim Überführen des Adsorbens aus dem Adsorbergehäuse 1A über die Schlauchleitungen 6 und 7 in die beiden Leerbeutel 3, 4. Die in dem Adsorbergehäuse 1A vorgesehenen Filtersiebe 1G, 1H verhindern, dass feste Stoffe in die Leerbeutel gelangen.

Nachdem das Adsorbergehäuse vollständig mit dem Adsorbens gefüllt ist, werden die Schlauchleitungen 5, 6 und 7 steril abgeschweißt, so dass der Adsorber von den Beuteln 2, 3, 4 getrennt ist. Es ist aber auch möglich, Absperrorgane zum Absperren der Schlauchleitungen vorzusehen.

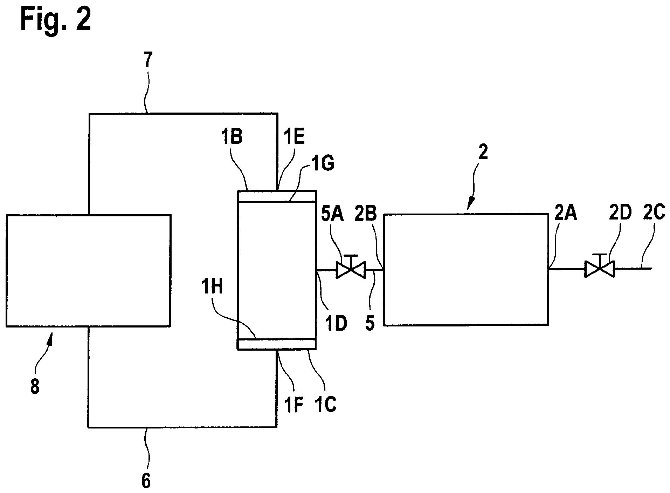

Fig. 2 zeigt eine alternative Ausführungsform der erfindungsgemäßen Anordnung, die sich von dem Ausführungsbeispiel von Fig. 1 nur dadurch unterscheidet, dass anstelle von zwei Leerbeuteln 3, 4 zur Aufnahme von Luft und/oder Flüssigkeit nur ein Leerbeutel 8 vorgesehen ist. Die einander entsprechenden Teile sind daher mit den gleichen Bezugszeichen versehen. Die beiden Schlauchleitungen 6, 7 sind bei dieser Ausführungsform an einander gegenüberliegenden Seiten an dem einzigen Leerbeutel 8 angeschlossen. In der Handhabung unterscheiden sich beide Ausführungsformen nur dadurch, dass Luft und/oder Flüssigkeit aus dem Adsorbergehäuse 1A nicht in zwei, sondern einen Leerbeutel 8 strömen.

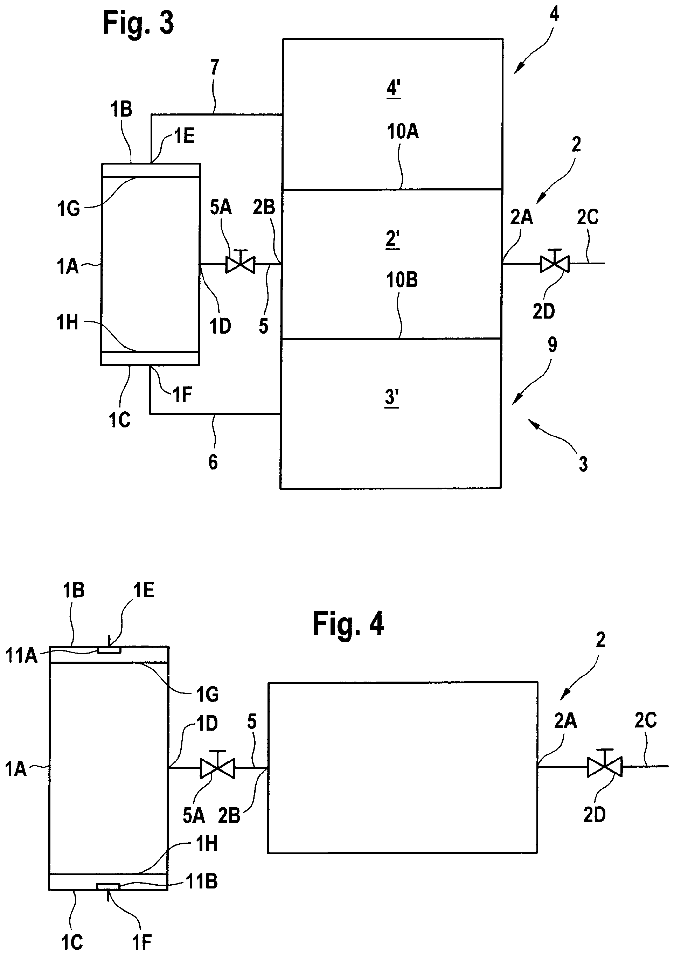

Fig. 3 zeigt eine weitere Ausführungsform der erfindungsgemäßen Anordnung, die sich von der Ausführungsform von Fig. 1 nur dadurch unterscheidet, dass der Beutel 2 zur Aufnahme des Adsorbens sowie die Beutel 3, 4 zur Aufnahme von Luft und/oder Flüssigkeit als Mehrkammer-Beutel 9 ausgebildet sind. Die einander entsprechenden Teile sind daher wieder mit den gleichen Bezugszeichen versehen. Der Dreikammer-Beutel 9 weist eine Kammer 2' für das Adsorbens und zwei Kammern 3' und 4' für Luft und/oder Flüssigkeit auf. Die drei Kammern 2', 3', 4' sind durch quer verlaufende Schweißnähte 10A, 10B voneinander getrennt. Der Dreikammer-Beutel 9 vereinfacht die Handhabung insofern, als sämtliche Beutel 2, 3, 4 eine Einheit bilden.

Fig. 4 zeigt eine weitere Ausführungsform der erfindungsgemäßen Anordnung, die sich von der Ausführungsform von Fig. 1 dadurch unterscheidet, dass die Beutel zur Aufnahme von Luft und/oder Flüssigkeit nicht vorhanden sind. Die einander entsprechenden Teile sind wieder mit den gleichen Bezugszeichen versehen. Bei dieser Ausführungsform sind die obere und die untere Entlüftungsöffnung 1E und 1F des Adsorbergehäuse 1A mit Sterilbarrieren, beispielsweise mit hydrophoben, d.h. für Gas durchlässigen, für Flüssigkeit aber nicht durchlässigen sterilen Membranen 11A, 11B verschlossen, die nur andeutungsweise dargestellt sind. Beim Befüllen des Adsorbergehäuses kann die Luft durch die Membranen 11A 11B entweichen, während aber die Flüssigkeit zurückgehalten wird. Daher kann bei dieser Ausführungsform überschüssige Flüssigkeit nicht abgeführt werden. Ansonsten wird die Ausführungsform von Fig. 4 wie die Ausführungsform von Fig. 1 gehandhabt.

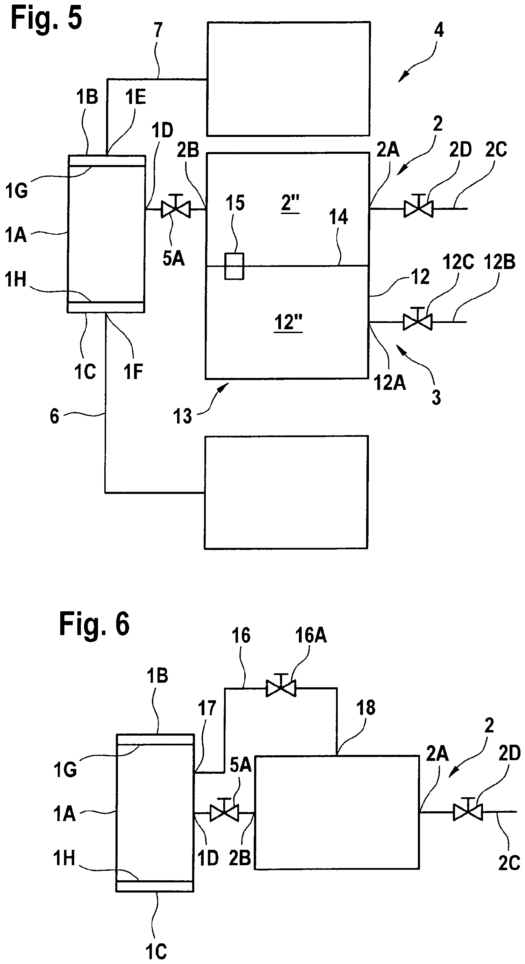

Fig. 5 zeigt eine weitere Ausführungsform der erfindungsgemäßen Anordnung, die sich von dem Ausführungsbeispiel von Fig. 1 dadurch unterscheidet, dass die Anordnung einen zusätzlichen Beutel 12 zur Aufnahme einer Spüllösung umfasst. Die einander entsprechenden Teile sind wieder mit den gleichen Bezugszeichen versehen. Der Beutel 2 zur Aufnahme des Adsorbens und der Beutel 12 zur Aufnahme der Spüllösung sind als Zweikammer-Beutel 13 ausgebildet, wobei die Kammer 2" für das Adsorbens und die Kammer 12" für die Spüllösung durch eine quer verlaufende Schweißnaht 14 voneinander getrennt sind. Die Kammer 12" für die Spüllösung weist einen Einlass 12A auf, an dem eine Schlauchleitung 12B angeschlossen ist, die mit einem Absperrorgan 12C verschlossen ist. Die Schlauchleitung 12B führt zu einem nicht dargstellten Reservoir für eine Spüllösung. Nach Öffnen des Absperrorgans 12C kann die Kammer 12" mit Spüllösung befüllt werden. Daraufhin wird das Absperrorgan 12C wieder geschlossen.

Die Ausführungsform von Fig. 5 wird wie die Ausführungsform von Fig. 1 gehandhabt. Allerdings erlaubt die Ausführungsform von Fig. 5, die entleerte Kammer 2" für das Adsorbens mit der in der Kammer 12" enthaltenden Spüllösung zu spülen. Hierzu wird nach dem Entleeren der Kammer 2" die Spüllösung aus der Kammer 13" in die Kammer 2" überführt. Dies kann dadurch erfolgen, dass eine Fluidverbindung zwischen der Kammer 12" und der Kammer 2" hergestellt wird. Vorzugsweise befindet sich in der Schweißnaht 14 ein von einem Abbrechteil 15 verschlossener Kanal, der durch Abbrechen des Abbrechteils geöffnet werden kann. Eine alternative Ausführungsform sieht vor, dass die Schweißnaht 14 zwischen den Kammern 2" und 12" aufreißbar ist. Da dem Fachmann Mehrkammer-Beutel, bei denen eine Fluidverbindung zwischen den einzelnen Kammern nachträglich hergestellt werden kann, allgemein bekannt sind, wird auf eine weitere Beschreibung des Zweikammer-Beutels verzichtet.

Nachdem die Kammer 2" mit der Spüllösung gespült ist, wird die Spüllösung mit der Restmenge an Adsorbens in das Adsorberghäuse 1A überführt. Erst dann wird das Absperrorgan 5A geschlossen. Die Schlauchleitungen 5, 6, 7 werden dann wieder steril von dem Adsorbergehäuse 1A abgeschweißt.

Bei einer alternativen Ausführungsform wird die Kammer 2" nicht mit einem Adsorbens in Form einer Suspension befüllt. Bei dieser Ausführungsform ist die Kammer 2" bereits mit einem pulverförmigen Adsorbens befüllt, das nach dem Überführen der Spüllösung aus der Kammer 12" in die Kammer 2" dann in gelöster Form in das Adsorbergehäuse 1A überführt wird.

Die Ausführungsform von Fig. 6 unterscheidet sich von der Ausführungsform von Fig. 1 dadurch, dass separate Beutel zur Aufnahme von Luft und/oder Fluid nicht vorgesehen sind. Die einander entsprechenden Teile sind wieder mit den gleichen Bezugszeichen versehen. Bei der Ausführungsform von Fig. 6 wird die in dem Adsorbergehäuse 1A befindliche Luft und/oder Flüssigkeit in den entleerten Beutel 2 überführt, der zuvor das Adsorbens aufgenommen hat. Hierzu ist eine Schlauchleitung 16 vorgesehen, die eine Entlüftungsöffnung 17 des Adsorbergehäuses 1A mit einer zweiten Einlassöffnung 18 des Beutels 2 verbindet. Die Schlauchleitung 16 ist mit einem Absperrorgan 16A, verschlossen. Beim Befüllen des Beutels 2 ist das Absperrorgan 16A geschlossen. Das Absperrorgan 16A wird zusammen mit dem Absperrorgan 5A geöffnet, und das Adsorbens aus dem Beutel 2 wird in das Adsorbergehäuse 1A überführt. Während das Adsorbens aus dem Beutel 2 in das Adsorbergehäuse 1A fließt, strömt die in dem Adsorbergehäuse verdrängte Luft und/oder Flüssigkeit über die Schlauchleitung 16A in das frei werdende Volumen des Beutels 2.

Für eine effektive Sterilisation sollte die Strahlendosis an allen Stellen mindestens ca. 25 kGy betragen, somit stellt die Strahlendosis von ca. 25 kGy idealerweise die Mindestdosis an der Oberfläche dar. Ab einer Strahlendosis von 50 kGy tritt eine erhebliche Schädigung auf die eine weitgehende Zerstörung der Aktivität des Adsorbermaterials bewirkt. Die Strahlendosis sollte in keiner Schichttiefe des Materials 50 kGy überschreiten. Bereits ab einer Strahlendosis von über 35 kGy tritt eine die Bindungsfähig verringernde Schädigung des Adsorbermaterials auf. Bevorzugt sollte die Strahlendosis daher einen Wert von 35 kGy im Material nicht überschreiten.

In Versuchen wurden Beutel mit den Außenmaßen von 300 x 260 mm bestrahlt, wobei die Füllhöhe der Beutel stets kleiner als 15 mm betrug. Im Mittel lag die Schichtdicke des bestrahlten Produkts bei ca. 12 mm. Die Bestrahlungsdosis betrug 27 und 28 kGy. An der Oberfläche des Produkts und unter dem Produkt wurden die folgenden Werte gemessen:

| Bestrahlungsdosis [kGy]: | Oberflächendosis [kGy] | Dosis unter dem Produkt [kGy] |
|---|---|---|
| 27,0 | 27,1 | 27,9 |
| 28,0 | 29,2 | 31,5 |
| 27,0 | 26,8 | 28,2 |
| 27,0 | 28,0 | 31,9 |
| 27,0 | 27,4 | 29,9 |
| 28,0 | 29,2 | 31,0 |
| 27,0 | 27,7 | 29,3 |

Es zeigt sich, dass sich die Strahlungsdosis innerhalb der dünnen Schicht kaum verändert. Die Unterschiede zu der erfindungsgemäßen Anordnung mit dem Beutel, in dem sich das Produkt in einer dünnen Schicht verteilt, werden anhand des folgenden Vergleichsversuchs deutlich.

Es wurden eine nass mit Adsorbermaterial gefüllte Adsorbersäule mit den Außenmaßen von 390 x 190 x 90 mm (Höhe = 90 mm), einer Masse von 358 g und einer Dichte von 0,054 g/cm³ zusammen mit einer Verpackung mit Elektronen einer Energie von 10MeV von ober und von unten mit einer Oberflächendosis auf der Verpackung von 24,6 kGy bestrahlt, wobei die Strahlungsdosis auf der Adsorbersäule, im Bereich der Oberseite der Säule, im Bereich der Unterseite der Säule und unter der Säule gemessen wurde. Auf der Säule wurde eine Dosis von 41,8 kGy und unter der Säule eine Dosis von 40,5 kGy gemessen, während in der Säule oben 51,5 kGy, in der Mitte der Säule 58,7 kGy und in der Säule unten 57,2 kGy gemessen wurden. Es zeigte sich, dass das Minimum im Produkt dabei 40,5 kGy betrug und das Maximum bei 58,7 kGy lag. Das Max/Min-Verhältnis beträgt demnach 58,7 / 40,5 = 1,45. In einer zweiten Messung wurden auf der Säule eine Dosis von 43,2 kGy und unter der Säule eine Dosis von 40,6 kGy gemessen, während in der Säule oben 55,9 kGy, in der Mitte der Säule 57,9 kGy und in der Säule unten 60,3 kGy gemessen wurden. Das Max/Min-Verhältnis beträgt demnach 60,3/40,6 = 1,49. Im Vergleich zu dem erfindungsgemäßen Beutel zeigt sich eine relativ starke Veränderung der Strahlungsdosis innerhalb der Schicht.

## Patentansprüche

1. Verfahren zum Sterilisieren eines Adsorbens, das in einem Behälter (1A) bereitgestellt werden soll, wobei das Adsorbens fließfähig oder rieselfähig ist, mit folgenden Verfahrensschritten:
Befüllen eines Behältnisses (2) mit dem zu sterilisierenden Adsorbens, wobei das Behältnis derart bemessen ist, dass sich das zu sterilisierende Adsorbens in dem Behältnis in einer dünnen Schicht mit einer Schichtdicke von bis zu 30 mm verteilt,
Bestrahlen des Behältnisses (2) mit einer energiereichen Strahlung mit einer Strahlendosis von mindestens 25 kGy und höchstens 50 kGy zum Sterilisieren des Adsorbens in dem Behältnis, und
Überführen des Adsorbens in den Behälter (1A), in dem das Adsorbens bereitgestellt werden soll,
wobei
das Behältnis (2) zum Sterilisieren und der Behälter (1A), in dem das Adsorbens bereitgestellt werden soll, ein geschlossenes System bilden, und
der Behälter, in dem das Adsorbens bereitgestellt werden soll, ein mit dem Adsorbens zu befüllendes Adsorbergehäuse (1A) ist.

2. Verfahren nach Anspruch 1, **dadurch gekennzeichnet, dass** das Behältnis zum Sterilisieren des Adsorbens ein Beutel (2) ist.

3. Verfahren nach Anspruch 1 oder 2, **dadurch gekennzeichnet, dass** das Adsorbens in das Adsorbergehäuse (1A) über eine das Behältnis (2) zum Sterilisieren des Adsorbens mit dem Adsorbergehäuse verbindende Leitung (5) überführt wird, wobei die Leitung nach dem Überführen des Adsorbens verschlossen wird.

4. Verfahren nach einem der Ansprüche 1 bis 3, **dadurch gekennzeichnet, dass** in dem Adsorbergehäuse (1A) befindliche Luft und/oder überschüssige Flüssigkeit aus dem Adsorbergehäuse abgeführt wird.

5. Verfahren nach Anspruch 4, **dadurch gekennzeichnet, dass** in dem Adsorbergehäuse (1A) befindliche Luft und/oder überschüssige Flüssigkeit in ein Behältnis (3, 4) überführt wird, das über eine Leitung (6, 7) mit dem Adsorbergehäuse (1A) verbunden ist.

6. Verfahren nach Anspruch 5, **dadurch gekennzeichnet, dass** das Behältnis zur Aufnahme der Luft und/oder Flüssigkeit ein Beutel (3, 4) ist.

7. Verfahren nach einem der Ansprüche 1 bis 6, **dadurch gekennzeichnet, dass** das Behältnis (2) zum Sterilisieren des Adsorbens mit einem flüssigen Adsorbens befüllt wird.

8. Verfahren nach einem der Ansprüche 1 bis 6, **dadurch gekennzeichnet, dass** das Behältnis (2) zum Sterilisieren des Adsorbens mit einem pulverförmigen Adsorbens befüllt wird.

9. Verfahren nach Anspruch 8, **dadurch gekennzeichnet, dass** eine Flüssigkeit in das Behältnis (2) zum Sterilisieren des Adsorbens überführt wird.

10. Verfahren nach einem der Ansprüche 7 bis 9, **dadurch gekennzeichnet, dass** nach dem Überführen des Adsorbens in das Adsorbergehäuse (1A) eine Spülflüssigkeit in das Behältnis (2) zum Sterilisieren des Adsorbens überführt wird, und nach dem Spülen des Behältnisses zum Sterilisieren des Adsorbens die Spülflüssigkeit in das Adsorbergehäuse (1A) überführt wird.

11. Anordnung zur Durchführung des Verfahrens nach einem der Ansprüche 1 bis 10 mit
einem Behältnis (2), das mit einem fließfähigen oder rieselfähigen Adsorbens befüllt ist, wobei das Behältnis (2) derart bemessen ist, dass sich das Adsorbens in dem Behältnis in einer dünnen Schicht mit einer Schichtdicke von bis zu 30 mm verteilt,
einem Adsorber (1), der ein mit dem Adsorbens zu befüllendes Adsorbergehäuse (1A) ist,
Mitteln zum Bestrahlen des Behältnisses (2) mit einer energiereichen Strahlung mit einer Strahlendosis von mindestens 25 kGy und höchstens 50 kGy zum Sterilisieren des Adsorbens in dem Behältnis, und
Mitteln (5) zur Herstellung einer Fluidverbindung zwischen dem mit einem Adsorbens befüllten Behältnis (2) und dem Adsorber (1), wobei das Behältnis und der Adsorber ein geschlossenes System bilden.

12. Anordnung nach Anspruch 11, **dadurch gekennzeichnet, dass** das mit einem Adsorbens befüllte Behältnis (2) ein Beutel (2) ist.

13. Anordnung nach Anspruch 12, **dadurch gekennzeichnet, dass** der Beutel (2) ein aus zwei übereinanderliegenden Folien oder ein aus einer Schlauchfolie gefertigter Folienbeutel ist.

14. Anordnung nach einem der Ansprüche 11 bis 13, **dadurch gekennzeichnet, dass** das mit einem Adsorbens befüllte Behältnis (2) einen Einlass (2A) zum Befüllen des Behältnisses mit einem Adsorbens aufweist, wobei der Einlass verschliessbar ist.

15. Anordnung nach einem der Ansprüche 11 bis 14, **dadurch gekennzeichnet, dass** die Mittel zur Herstellung einer Fluidverbindung zwischen dem mit einem Adsorbens befüllten Behältnis (2) und dem Adsorbergehäuse (1A) eine Schlauchleitung (5) aufweisen.

16. Anordnung nach Anspruch 15, **dadurch gekennzeichnet, dass** die Schlauchleitung (5) zur Verbindung des mit einem Adsorbens befüllten Behältnisses (2) mit dem Adsorbergehäuse (1A) ein Absperrorgan (5A) aufweist.

17. Anordnung nach einem der Ansprüche 11 bis 16, **dadurch gekennzeichnet, dass** das Adsorbergehäuse (1A) mindestens eine Entlüftungsöffnung (1E, 1F) aufweist, die mit einer sterilen Barriere (12A, 12B), insbesondere einer hydrophoben sterilen Membran verschlossen ist.

18. Anordnung nach einem der Ansprüche 11 bis 16, **dadurch gekennzeichnet, dass** das Adsorbergehäuse (1A) mindestens eine Entlüftungsöffnung (17) aufweist, an der eine zu dem mit einem Adsorbens befüllten Behältnis (2) führende Schlauchleitung (16) angeschlossen ist.

19. Anordnung nach einem der Ansprüche 11 bis 16, **dadurch gekennzeichnet, dass** die Anordnung ein Behältnis (8) zur Aufnahme von in dem Adsorbergehäuse (1A) befindlicher Luft und/oder überschüssiger Flüssigkeit und Mittel (6, 7) zum Überführen von Luft und/oder Flüssigkeit zwischen dem Adsorbergehäuse (1A) und dem Behältnis (8) zur Aufnahme von Luft und/oder Flüssigkeit aufweist.

20. Anordnung nach Anspruch 19, **dadurch gekennzeichnet, dass** die Mittel zum Überführen der Luft und/oder Flüssigkeit zwischen dem Adsorbergehäuse (1A) und dem Behältnis (3) zur Aufnahme von Luft und/oder Flüssigkeit eine erste und eine zweite Schlauchleitung (6, 7) aufweisen, wobei das eine Ende der ersten Schlauchleitung (6) an eine erste Entlüftungsöffnung (1F) des Adsorbergehäuses (1A) und das andere Ende der ersten Schlauchleitung (6) an das Behältnis (3) und das eine Ende der zweiten Schlauchleitung (7) an eine zweite Entlüftungsöffnung (1E) des Adsorbergehäuse (1A) und das andere Ende der zweiten Schlauchleitung (7) an das Behältnis (3) angeschlossen sind, wobei die erste und zweite Entlüftungsöffnung (1F, 1E) des Adsorbergehäuses (1A) an einander gegenüberliegenden Seiten des Adsorbergehäuses angeordnet sind.

21. Anordnung nach Anspruch 20, **dadurch gekennzeichnet, dass** die erste Entlüftungsöffnung (1E) an der Unterseite und die zweite Entlüftungsöffnung (1F) an der Oberseite des Adsorbergehäuses (1A) angeordnet ist.

22. Anordnung nach einem der Ansprüche 11 bis 16, **dadurch gekennzeichnet, dass** die Anordnung ein erstes und ein zweites Behältnis (3, 4) zur Aufnahme von in dem Adsorbergehäuse (1A) befindlicher Luft und/oder überschüssiger Flüssigkeit und Mittel (6, 7) zum Überführen von Luft und/oder Flüssigkeit zwischen dem Adsorbergehäuse (1A) einerseits und dem ersten bzw. zweiten Behältnis (3, 4) zur Aufnahme von Luft und/oder Flüssigkeit andererseits aufweist.

23. Anordnung nach Anspruch 22, **dadurch gekennzeichnet, dass** die Mittel zum Überführen der Luft und/oder Flüssigkeit eine erste und eine zweite Schlauchleitung (6, 7) aufweisen, wobei das eine Ende der ersten Schlauchleitung (6) an eine erste Entlüftungsöffnung (1F) des Adsorbergehäuses (1A) und das andere Ende der ersten Schlauchleitung (6) an das erste Behältnis (3) angeschlossen ist, und das eine Ende der zweiten Schlauchleitung (7) an eine zweite Entlüftungsöffnung (1E) des Adsorbergehäuses (1A) und das andere Ende der zweiten Schlauchleitung (7) an das zweite Behältnis (4) angeschlossen ist, wobei die erste und zweite Entlüftungsöffnung (1F, 1E) des Adsorbergehäuses (1A) an einander gegenüberliegenden Seiten des Adsorbergehäuses angeordnet sind.

24. Anordnung nach Anspruch 23, **dadurch gekennzeichnet, dass** die erste Entlüftungsöffnung (1F) an der Unterseite und die zweite Entlüftungsöffnung (1E) an der Oberseite des Adsorbergehäuses (1A) angeordnet ist.

25. Anordnung nach einem der Ansprüche 11 bis 24, **dadurch gekennzeichnet, dass** das Adsorbergehäuse (1A) ein hohlzylindrisches Gehäuse ist.

26. Anordnung nach einem der Ansprüche 18 bis 25, **dadurch gekennzeichnet, dass** das Behältnis zur Aufnahme von Luft und/oder Flüssigkeit ein Beutel (3, 4) ist.

## Claims

1. Method of sterilising an adsorbent being flowable or pourable, which is to be made available in a container (1A), having the following method steps:
filling of a containing means (2) with the adsorbent to be sterilised, the containing means being of a size such that the adsorbent to be sterilised distributes itself in the containing means in a thin film or layer having a thickness of up to 30 mm,
irradiation of the containing means with high-energy radiation having an intensity of at least 25 kGy and not exceeding 50 kGy to sterilise the adsorbent in the containing means, and
transfer of the adsorbent into a container (1A) in which the adsorbent is to be made available,
the containing means (2) for sterilisation and the container (1A) in which the adsorbent is to be made available forming a closed system, and
the container, in which the adsorbent is to be made available, is an adsorber housing (1A) which is to be filled with the adsorbent.

2. Method according to claim 1, **characterised in that** the containing means for the sterilisation of the adsorbent is a bag (2).

3. Method according to claim 1 or 2, **characterised in that** the adsorbent is transferred into the adsorber housing (1A) by means of a line (5) which connects the containing means (2) for the sterilisation of the adsorbent to the adsorber housing, the line being closed off after the transfer of the adsorbent.

4. Method according to one of claims 1 to 3, **characterised in that** air and/or excess liquid/fluid situated in the adsorber housing (1A) is fed away out of the adsorber housing.

5. Method according to claim 4, **characterised in that** air and/or excess liquid/fluid situated in the adsorber housing (1A) is transferred into a containing means (3, 4) which is connected to the adsorber housing (1A) via a line (6, 7).

6. Method according to claim 5, **characterised in that** the containing means for receiving air and/or liquid/fluid is a bag (3, 4).

7. Method according to one of claims 1 to 6, **characterised in that** the containing means (2) for the sterilisation of the adsorbent is filled with a liquid/fluid adsorbent.

8. Method according to one of claims 1 to 6, **characterised in that** the containing means (2) for the sterilisation of the adsorbent is filled with an adsorbent in powder form.

9. Method according to claim 8, **characterised in that** a liquid/fluid is transferred into the containing means (2) for the sterilisation of the adsorbent.

10. Method according to one of claims 7 to 9, **characterised in that** a flushing liquid/fluid is transferred into the containing means (2) for the sterilisation of the adsorbent once the adsorbent has been transferred into the adsorber housing (1A), and after the containing means for the sterilisation of the adsorbent has been flushed the flushing liquid/fluid is transferred into the adsorber housing (1A).

11. Arrangement for performing the method of one of claims 1 to 10 having
a containing means (2) which is filled with a flowable or pourable adsorbent, which containing means is of a size such that the adsorbent is able to distribute itself in the containing means in a thin film or layer having a thickness of up to 30 mm,
an adsorber (1), which is an adsorber housing (1A) which is to be filled with the adsorbent,
means for irradiation of the containing means (2) with high-energy radiation having an intensity of at least 25 kGy and not exceeding 50 kGy to sterilise the adsorbent in the containing means, and
means (5) for making a connection for fluid between the containing means (2) to be filled with an adsorbent and the adsorber (1), the containing means and the adsorber forming a closed system.

12. Arrangement according to claim 11, **characterised in that** the containing means to be filled with an adsorbent is a bag (2).

13. Arrangement according to claim 12, **characterised in that** the bag (2) is a film bag which is produced from two films situated one above the other or from a tube of film.

14. Arrangement according to one of claims 11 to 13, **characterised in that** the containing means (2) to be filled with an adsorbent has an inlet (2A) for filling the containing means with an adsorbent, the inlet being able to be closed off.

15. Arrangement according to one of claims 11 to 14, **characterised in that** the means for making a connection for fluid between the containing means (2) to be filled with an adsorbent and the adsorber housing (1A) have a flexible line (5).

16. Arrangement according to claim 15, **characterised in that** the flexible line (5) for connecting the containing means (2) to be filled with an adsorbent to the adsorber housing (1A) has a shut-off device (5A).

17. Arrangement according to one of claims 11 to 16, **characterised in that** the adsorber housing (1A) has at least one venting opening (1E, 1F) which is closed off by a sterile barrier (12A, 12B) and in particular by a hydrophobic sterile membrane.

18. Arrangement according to one of claims 11 to 16, **characterised in that** the adsorber housing (1A) has at least one venting opening (17) to which is connected a flexible line (16) which runs to the containing means (2) to be filled with an adsorbent.

19. Arrangement according to one of claims 11 to 16, **characterised in that** the arrangement has a containing means (8) for receiving air and/or excess liquid/fluid which is situated in the adsorber housing (1A), and means (6, 7) for transferring air and/or liquid/fluid between the adsorber housing (1A) and the containing means (8) for receiving air and/or liquid/fluid.

20. Arrangement according to claim 19, **characterised in that** the means for transferring air and/or liquid/fluid between the adsorber housing (1A) and the containing means (3) for receiving air and/or liquid/fluid have a first and a second flexible line (6, 7), one end of the first flexible line (6) being connected to a first venting opening (1F) in the adsorber housing (1A) and the other end of the first flexible line (6) being connected to the containing means (3), and one end of the second flexible line (7) being connected to a second venting opening (1E) in the adsorber housing (1A) and the other end of the second flexible line (7) being connected to the containing means (3), the first and second venting openings (1F, 1E) in the adsorber housing (1A) being arranged at opposite ends or sides of the adsorber housing from one another.

21. Arrangement according to claim 20, **characterised in that** the first venting opening (1E) is arranged at the bottom end of the adsorber housing (1A) and the second venting opening (1F) is arranged at the top end of the adsorber housing (1A).

22. Arrangement according to one of claims 11 to 16, **characterised in that** the arrangement has a first and a second containing means (3, 4) for receiving air and/or excess liquid/fluid which is situated in the adsorber housing (1A), and means (6, 7) for transferring air and/or liquid/fluid between the adsorber housing (1A) on the one hand and, respectively, the first and second containing means (3, 4) for receiving air and/or liquid/fluid on the other hand.

23. Arrangement according to claim 22, **characterised in that** the means for transferring air and/or liquid/fluid have a first and a second flexible line (6, 7), one end of the first flexible line (6) being connected to a first venting opening (1F) in the adsorber housing (1A) and the other end of the first flexible line (6) being connected to the first containing means (3), and one end of the second flexible line (7) being connected to a second venting opening (1E) in the adsorber housing (1A) and the other end of the second flexible line (7) being connected to the second containing means (4), the first and second venting openings (1F, 1E) in the adsorber housing (1A) being arranged at opposite ends or sides of the adsorber housing from one another.

24. Arrangement according to claim 23, **characterised in that** the first venting opening (1F) is arranged at the bottom end of the adsorber housing (1A) and the second venting opening (1E) is arranged at the top end of the adsorber housing (1A).

25. Arrangement according to one of claims 11 to 24, **characterised in that** the adsorber housing (1A) is a hollow cylindrical housing.

26. Arrangement according to one of claims 18 to 25, **characterised in that** the containing means for receiving air and/or liquid/fluid is a bag (3, 4).

## Revendications

1. Procédé de stérilisation d'un adsorbant, qui doit être mis à disposition dans un récipient (1A), dans lequel l'adsorbant est apte à s'écouler ou à se répandre, comprenant les étapes suivantes du procédé :
remplissage d'un réceptacle (2) avec l'adsorbant à stériliser, dans lequel le réceptacle est dimensionné de telle sorte que l'adsorbant à stériliser soit réparti en une couche mince dans le réceptacle, avec une épaisseur de couche allant jusqu'à 30 mm,
irradiation du réceptacle (2) par un rayonnement de haute énergie avec une dose de rayonnement d'au moins 25 kGy et au plus de 50 kGy pour la stérilisation de l'adsorbant dans le réceptacle, et
transfert de l'adsorbant dans le récipient (1A) dans lequel l'adsorbant doit être mis à disposition,
dans lequel
le réceptacle (2) pour la stérilisation et le récipient (1A) dans lequel l'adsorbant doit être mis à disposition forment un système fermé, et
le récipient dans lequel l'adsorbant doit être mis à disposition est un logement d'adsorbeur (1A) à remplir par l'adsorbant.

2. Procédé selon la revendication 1, **caractérisé en ce que** le réceptacle pour la stérilisation de l'adsorbant est une poche (2).

3. Procédé selon la revendication 1 ou 2, **caractérisé en ce que** l'adsorbant dans le logement d'adsorbeur (1A) est transféré par une conduite (5) reliant le réceptacle (2) pour la stérilisation de l'adsorbant au logement de l'adsorbeur, dans lequel la conduite est fermée après le transfert de l'adsorbant.

4. Procédé selon l'une des revendications 1 à 3, **caractérisé en ce que** de l'air et/ou du liquide excédentaire se trouvant dans le logement d'adsorbeur (1A) est évacué du logement d'adsorbeur.

5. Procédé selon la revendication 4, **caractérisé en ce que** de l'air et/ou du liquide excédentaire se trouvant dans le logement d'adsorbeur (1A) est transféré dans un réceptacle (3, 4) qui est relié par une conduite (6, 7) au logement d'adsorbeur (1A).

6. Procédé selon la revendication 5, **caractérisé en ce que** le réceptacle pour la réception de l'air et/ou du liquide est une poche (3, 4).

7. Procédé selon l'une des revendications 1 à 6, **caractérisé en ce que** le réceptacle (2) pour la stérilisation de l'adsorbant est rempli d'un adsorbant liquide.

8. Procédé selon l'une des revendications 1 à 6, **caractérisé en ce que** le réceptacle (2) pour la stérilisation de l'adsorbant est rempli d'un adsorbant pulvérulent.

9. Procédé selon la revendication 8, **caractérisé en ce qu'**un liquide est transféré dans le réceptacle (2) pour la stérilisation de l'adsorbant.

10. Procédé selon l'une des revendications 7 à 9, **caractérisé en ce qu'**après le transfert de l'adsorbant dans le logement d'adsorbeur (1A), un liquide de rinçage est transféré dans le réceptacle (2) pour la stérilisation de l'adsorbant et après le rinçage du réceptacle pour la stérilisation de l'adsorbant, le liquide de rinçage est transféré dans le logement d'adsorbeur (1A).

11. Arrangement pour la mise en oeuvre du procédé selon l'une des revendications 1 à 10, comportant :
un réceptacle (2) qui est rempli d'un adsorbant apte à s'écouler ou à se répandre, dans lequel le réceptacle (2) est dimensionné de telle sorte que l'adsorbant dans le réceptacle soit réparti en une couche mince avec une épaisseur de couche allant jusqu'à 30 mm,
un adsorbeur (1) qui est un logement d'adsorbeur à remplir par l'adsorbant (1A),
des moyens pour irradier le réceptacle (2) avec un rayonnement de haute énergie avec une dose de rayonnement d'au moins 25 kGy et au plus de 50 kGy pour la stérilisation de l'adsorbant dans le réceptacle, et
des moyens (5) pour produire une liaison fluidique entre le réceptacle (2) pour la réception de l'adsorbant et l'adsorbeur (1), dans lequel le réceptacle et l'adsorbeur forment un système fermé.

12. Arrangement selon la revendication 11, **caractérisé en ce que** le réceptacle (2) rempli par un adsorbant est une poche (2).

13. Arrangement selon la revendication 12, **caractérisé en ce que** la poche (2) est une poche de type film fabriquée à partir de deux films superposés ou à partir d'un film tubulaire.

14. Arrangement selon l'une des revendications 11 à 13, **caractérisé en ce que** le réceptacle (2) rempli d'un adsorbant présente une entrée (2A) pour le remplissage du réceptacle avec un adsorbant, l'entrée étant apte à être fermée.

15. Arrangement selon l'une des revendications 11 à 14, **caractérisé en ce que** les moyens pour produire une liaison fluidique entre le réceptacle (2) rempli d'un adsorbant et le logement d'adsorbeur (1A) présentent une conduite tubulaire (5).

16. Arrangement selon la revendication 15, **caractérisé en ce que** la conduite tubulaire (5) pour la liaison du réceptacle (2) rempli d'un adsorbant au logement d'adsorbeur (1A) présente un organe de blocage (5A).

17. Arrangement selon l'une des revendications 11 à 16, **caractérisé en ce que** le logement d'adsorbeur (1A) présente au moins une ouverture de ventilation (1E, 1F) qui est fermée avec une barrière stérile (12A, 12B), en particulier une membrane stérile hydrophobe.

18. Arrangement selon l'une des revendications 11 à 16, **caractérisé en ce que** le logement d'adsorbeur (1A) présente au moins une ouverture de ventilation (17) à laquelle est raccordée une conduite tubulaire conduisant au réceptacle (2) rempli d'un adsorbant (16).

19. Arrangement selon l'une des revendications 11 à 16, **caractérisé en ce que** l'arrangement présente un réceptacle (8) pour la réception de l'air et/ou du liquide excédentaire se trouvant dans le logement d'adsorbeur (1A) et des moyens (6, 7) pour le transfert d'air et/ou de liquide entre le logement d'adsorbeur (1A) et le réceptacle (8) pour la réception de l'air et/ou du liquide.

20. Arrangement selon la revendication 19, **caractérisé en ce que** les moyens pour le transfert de l'air et/ou du liquide entre le logement d'adsorbeur (1A) et le réceptacle (3) pour la réception d'air et/ou de liquide présentent une première et une deuxième conduites tubulaires (6, 7), dans lequel une extrémité de la première conduite tubulaire (6) est raccordée à une première ouverture de ventilation (1F) du logement d'adsorbeur (1A) et l'autre extrémité de la première conduite tubulaire (6) est raccordée au réceptacle (3) et une extrémité de la deuxième conduite tubulaire (7) est raccordée à une deuxième ouverture de ventilation (1E) du logement d'adsorbeur (1A) et l'autre extrémité de la deuxième conduite tubulaire (7) est raccordée au réceptacle (3), dans lequel les première et deuxième ouvertures de ventilation (1F, 1E) du logement d'adsorbeur (1A) sont disposées sur des côtés opposés du logement d'adsorbeur.

21. Arrangement selon la revendication 20, **caractérisé en ce que** la première ouverture de ventilation (1E) est disposée sur la face inférieure et la deuxième ouverture de ventilation (1F) est disposée sur la face supérieure du logement d'adsorbeur (1A).

22. Arrangement selon l'une des revendications 11 à 16, **caractérisé en ce que** l'arrangement présente un premier et un deuxième réceptacles (3, 4) pour la réception de l'air et/ou du liquide excédentaire se trouvant dans le logement d'adsorbeur (1A) et des moyens (6, 7) pour le transfert d'air et/ou de liquide entre le logement d'adsorbeur (1A) d'une part et le premier ou deuxième réceptacle (3, 4) pour la réception de l'air et/ou du liquide, d'autre part.

23. Arrangement selon la revendication 22, **caractérisé en ce que** les moyens pour le transfert de l'air et/ou du liquide présentent une première et une deuxième conduites tubulaires (6, 7), dans lequel une extrémité de la première conduite tubulaire (6) est raccordée à une première ouverture de ventilation (1F) du logement d'adsorbeur (1A) et l'autre extrémité de la première conduite tubulaire (6) est raccordée au premier réceptacle (3) et une extrémité de la deuxième conduite tubulaire (7) est raccordée à une deuxième ouverture de ventilation (1E) du logement d'adsorbeur (1A) et l'autre extrémité de la deuxième conduite tubulaire (7) est raccordée au deuxième réceptacle (4), dans lequel les première et deuxième ouvertures de ventilation (1F, 1E) du logement d'adsorbeur (1A) sont disposées sur des côtés opposés du logement d'adsorbeur.

24. Arrangement selon la revendication 23, **caractérisé en ce que** la première ouverture de ventilation (1F) est disposée sur la face inférieure et la deuxième ouverture de ventilation (1E) est disposée sur la face supérieure du logement d'adsorbeur (1A).

25. Arrangement selon l'une des revendications 11 à 24, **caractérisé en ce que** le logement d'adsorbeur (1A) est un logement cylindrique creux.

26. Arrangement selon l'une des revendications 18 à 25, **caractérisé en ce que** le réceptacle pour la réception d'air et/ou de liquide est une poche (3, 4).
